# EUROPEAN PATENT SPECIFICATION

(11) **EP 1 485 048 B1**
(45) Date of publication and mention of the grant of the patent: **01.06.2016**
(21) Application number: 03714706.3
(22) Date of filing: 11.03.2003
(51) Int. Cl.: A61F 5/448, A61F 5/445

(54) **SOFT CONVEX ADHESIVE WAFER**
WEICHE KONVEXE KLEBENDE SCHEIBE
PLAQUETTE ADHESIVE CONVEXE SOUPLE

(30) Priority: 11.03.2002 US 95223
(43) Date of publication of application: 15.12.2004
(60) Divisional application: 10183729.2
(73) Proprietor: Dansac A/S, 3480 Fredensborg (DK)
(72) Inventor: LEISE, Walter, F., Jr., Lindenhurst, IL 60046 (US); BOTTEN, Ronald, S., Gurnee, IL 60031 (US); PEDERSEN, Ole, 2000 Frederiksberg (DK); GLAESER, Christopher, T., Grayslake, IL 60030 (US); NIELSEN, Kristoffer, Stubtoft, 3250 Gilleleje (DK)
(74) Representative: Høiberg A/S
(86) International application number: PCT/DK2003/000148
(87) International publication number: WO 2003/075808

(56) References cited:
- EP-A- 0 800 804
- EP-A- 1 163 892
- EP-A2- 0 264 299
- EP-A2- 0 806 210
- US-A- 5 716 475
- US-A- 6 093 276

## Description

The present invention relates to a wafer for adhesively attaching an ostomy appliance to a peristomal skin surface surrounding a stoma of a wearer of said appliance.

A wafer according to the preamble of claim 1 is known from EP 0 806 210. Other wafers of similar kind are known from EP 1 163 892, US 5,716,475, and US 6 093 276.

Within the field of ileostomy, colostomy and urostomy appliances, it is well known that the adhesive wafer should perform a series of important functions such as ensuring secure fastening of the appliance to the skin of the wearer, ensuring a secure sealing function between the exuded stomal fluids and the skin and stoma of the wearer, providing a maximum of comfort and security to said wearer as well as prolonging the wear time of the wafer for economic and practical reasons.

Naturally, because of the inherent severe medical, social, economical and personal problems related to the need for use of an ostomy appliance, any improvement that addresses said problems is an important factor in the quality of life of such wearers and therefore in the consequent health and psychological aspects for said wearer.

Any appreciable improvement of such appliances is therefore of great importance to the increasing number of such wearers.

A large proportion of the problems in connection with ostomy appliances are related to the topography of the peristomal surface surrounding the stoma and of the stoma itself.

On account of a variety of reasons such as initial surgery results, weight increase, muscle relaxation and use of pressure rings in connection with rigid convex wafers or face plates, a groove or moat around the stoma is initially present or develops in a large number of such wearers. These moats can have different shapes, depths and widths and result in difficulties in applying said appliances in a manner that satisfies the requirements as to comfort, adhesion and protection of sensitive skin areas from contact with stomal fluids.

The stoma may protrude more or less, and if the protrusion is small or non-existent, it is necessary to press the adhesive wafer in towards the skin of the wearer so as to cause the stoma to protrude out by pressing the peristomal skin surface inwards. This is usually done with a convex combination of a wafer with substantially uniform thickness deformed to a convex distal bodyside configuration by a stiff convex ring that is attached to a belt so as to exert pressure on the wafer to press the convex bodyside surface thereof caused by the convex ring into the peristomal skin surface of the wearer.

Such a stiff convex ring causes discomfort, is relatively expensive and causes deformation of the peristomal tissue so that formation of a groove or moat around the stoma ensues. This again leads to difficulties in proper sealing around the stoma with ensuing problems and leakage of stomal fluids leading to sores and shorter wear time for the appliance.

To overcome these problems it is known to fill out the moat and seal against the stoma by applying a paste or a separate ring of deformable skin barrier material to the stoma and peristomal skin surface prior to applying the wafer. This is an operation that requires dexterity and is not easy to perform correctly and requires sometimes difficult separate removal of the paste or ring when the wafer is to be replaced.

European patent application No. EP 1 163 892 discloses a deformable ring of skin barrier material that is adhered to the proximal surface of an essentially uniformly thick wafer after being deformed to fit the exterior circumference of the stoma. This solution entails a relatively complicated operation that requires dexterity and therefore is difficult to apply correctly. Because of the various separate elements constituting the combined wafer and ring, it is also relatively expensive.

International patent application No. WO 0053133 discloses a relatively thin adhesive layer of skin barrier material having a thickness of between approximately 0.9 mm and 1.2 mm and shaped to a form a central dome surrounding a stoma receiving aperture and surrounded by a substantially planar peripheral portion. The hollow proximally convex and distally concave dome is deformable because of the relatively thin unreinforced configuration thereof. The deformability of the dome entails that no appreciable pressure is applied to the peristomal surface and the stoma itself. Furthermore, the ability of the dome surrounding the stoma to absorb moisture without losing the adhesive property of the dome material is relatively limited due to the relatively slight thickness of the adhesive layer.

It is a main object of the present invention to provide a wafer of the type indicated that may fit into a moat or groove in the peristomal skin surface surrounding a stoma in a manner providing a certain pressure on said peristomal surface and lateral support pressure on said stoma while at the same time having a relatively large capability of absorbing moisture, being easy to apply and remove and relatively cheap to manufacture.

According to the invention this object is achieved by a wafer according to claim 1.

Advantageously, said peripheral portion has a width of at least 10 mm, preferably at least 12 mm and most preferably at least 15 mm, and the thickness of said peripheral portion is at least 0.4 mm, preferably at least 0,5 mm, more preferably at least 0.6 mm, even more preferably at least 0.7 mm and most preferably at least 0.8 mm.

Hereby, the secure application of the central portion of the bodyside surface of the adhesive layer into a moat surrounding the stoma is ensured in a manner giving a good sealing effect and with exertion of a certain pressure against said peristomal surface and against said stoma.

In the currently preferred embodiment of a wafer according to the invention, said central portion is annular, and said aperture is substantially circular and concentric with said annular central portion.

So as to ensure that good lateral support of the stoma can be obtained, said central portion should have a radial width of at least 6 mm, preferably at least 7 mm and most preferably larger than or equal to 7.5 mm.

So as to ensure that the central portion may fit well around the stoma said annular portion should have a radial width of less than 11 mm, preferably less than 10 mm, most preferably less than or equal to 9.5 mm.

In the currently preferred embodiment of a wafer according to the invention, said central portion has a substantially uniform thickness.

However, so as to accommodate more irregular moat configurations, alternative embodiments are useful where said central portion has a thickness that varies in the circumferential direction and/or in the radial direction.

In another aspect, the present invention relates to a set of at least two wafers for adhesively attaching an ostomy appliance to a peristomal skin surface surrounding a stoma of a wearer of said appliance, each wafer of said set comprising an integrally moulded adhesive layer consisting of a moisture absorbing and swellable skin barrier material and having a substantially planar distal surface covered by a flexible backing layer for attaching said wafers to a collecting bag and a proximal bodyside surface that is contoured such that said adhesive layer has a relatively thin peripheral portion and a relatively thick central portion surrounding an aperture for receiving said stoma, said central portion being distributed substantially uniformly around said aperture and having a maximum thickness which is at least 2.5 mm larger than the maximum thickness of said peripheral portion, the individual wafers of said set differing from one another in that the topography of the surface of said central portion is different and optionally in that the size and/or shape of said stoma receiving aperture is different.

It is here described a method of attaching an ostomy appliance to a peristomal skin surface surrounding a stoma of a wearer of said appliance, the method comprising the following steps:
- providing a set of at least two wafers for adhesively attaching said ostomy appliance to said peristomal skin surface, each wafer of said set comprising an adhesive layer consisting of a moisture absorbing and swellable skin barrier material and having a substantially planar distal surface covered by a flexible backing layer for attaching said wafers to a collecting bag and a proximal bodyside surface that is contoured such that said adhesive layer has a relatively thin peripheral portion and a relatively thick central portion surrounding an aperture for receiving said stoma, said central portion being distributed substantially uniformly around said aperture and having a maximum thickness which is at least 2.5 mm larger than the maximum thickness of said peripheral portion, the individual wafers of said set differing from one another in that the topography of the surface of said central portion is different,
- evaluating the topography of said persitomal surface and said stoma,
- selecting a specific wafer from among the wafers in said set based on said topographical evaluation such that the topography of the surface of said central portion of said specific wafer has the best fit to said topography of said peristomal surface and said stoma, and
- locating and adhesively attaching said selected specific wafer on said peristomal surface such that said best fit is exploited.

Hereby, a particularly good fit may be achieved between the peristomal surface and stoma of a specific wearer of an ostomy appliance and the wafer for adhering said appliance. The added cost of producing, stocking and dispensing a set of differently configured wafers will be more than compensated by the added comfort, wearing time, sealing properties and feeling of security achieved by being able to apply the wafer best suited to the requirements of a specific wearer.

In a yet further aspect, the present invention relates to a method of producing a wafer according to claim 21.

Hereby, a wafer is provided having as good a fit to the peristomal surface and stoma of a specific wearer as possible. This will maximize the comfort, sealing properties, wearing time and security for the wearer. In view of the impact on wearers of ostomy appliances of having to wear such an appliance as regards the day-to-day quality of life, the added cost of such customized wafers is fully justified. If the total costs of wearing ostomy appliances including hospital treatment for skin and stoma lesions, frequency of replacement, sick leave and so on are taken into account, such added costs for customization may be more than compensated.

A simple embodiment of the method according to the invention comprises the step of producing the representation by making a cast or impression of said peristomal surface and stoma.

The currently preferred method according to the invention comprises the steps of:
- producing said representation in digital form by scanning said peristomal surface and stoma or by scanning said cast or impression,
- utilizing said digital representation to produce a mould, and
- moulding said wafer in said mould.

Hereby, the specific wearer of the ostomy appliance is not inconvenienced more than necessary, and no actual physical contact is to be endured as when making a cast or impression. Furthermore, the digital representation may be stored, communicated and amended in many ways.

Preferably, said digital representation is obtained by scanning with x-rays, light rays, MRI, CAT-scan or ultrasound.

It is also here described a method of attaching an ostomy appliance to a peristomal skin surface surrounding a stoma of a specific wearer of said appliance by means of a wafer comprising an adhesive layer consisting of a moisture absorbing and swellable skin barrier material and having a distal surface for attaching said wafer to a collecting bag and a proximal bodyside surface for adhering to said peristomal skin surface, the method comprising the following steps:
- manufacturing said wafer by means of a method as described in the preceding paragraphs, said representation being a representation of the topography of the peristomal surface of said specific wearer, and
- locating and adhesively attaching said wafer on said peristomal surface such that a close fit of said bodyside surface of said wafer to said peristomal surface and stoma is achieved.

The present invention relates to a wafer for adhesively attaching an ostomy appliance to a peristomal skin surface surrounding a stoma of a wearer of said appliance, said wafer comprising an integrally moulded adhesive layer consisting of a moisture absorbing and swellable skin barrier material and having a distal surface covered by a flexible backing layer for attaching said wafer to a collecting bag, said flexible backing layer extending at least to the outer periphery of said adhesive layer, the shape of said outer periphery consisting of outwardly convex or substantially rectilinear portions and at least one outwardly concave portion or indentation.

Such an outwardly concave portion or indentation allows a gripping portion of the backing layer to be formed by configuring the backing layer periphery without said indentation such that an area of said backing layer corresponding to the area of said indentation is not adhered to the persitomal skin surface by the adhesive layer and can be gripped for peeling off the adhesive layer from said skin surface when the wafer is to be replaced.

In the currently preferred embodiment of a wafer according to the invention, said outer periphery is generally rectangular and an outwardly concave portion or indentation is located at one of the four corners of said rectangular periphery, preferably at all four corners thereof.

It is also here described a mould for producing a wafer for adhesively attaching an ostomy appliance to a peristomal skin surface surrounding a stoma of a wearer of said appliance, said wafer comprising an adhesive layer consisting of a moisture absorbing and swellable skin barrier material and having a sharply defined outer periphery, the mould comprising a first part and a second part adapted for having moulding surfaces thereof pressed against one another such that mould hollows in one or both said surfaces together define the configuration of said adhesive layer, at least one periphery defining body for defining said outer periphery being arranged in a groove in a surface of one of the first or second part such that said body is displaceable in the direction towards the other part.

Hereby, a simple means is provided for ensuring a sharply defined periphery of the adhesive layer such that soiling or adhesion of the clothes or bedclothes of a wearer is avoided.

In the currently preferred mould, a biasing means is arranged in said groove in one of said first and second parts for biasing said body in said direction towards the other part when said parts are pressed together, and said body is annular and comprises outwardly convex portions and/or substantially rectilinear portions and outwardly concave portions or indentations.

In a final aspect, the present invention relates to a method of laminating a wafer according to any of the claims 1-17.

Hereby, the release sheet is applied and laminated to the bodyside surface of the adhesive layer in a manner allowing short moulding time in the second mould hollow. If the release sheet were formed and laminated to the adhesive layer in the second mould hollow this would require a much longer moulding time and consequent lower production rate. The much longer moulding time in such case is owing to formation of wrinkles in the release sheet, which requires a long moulding pressure duration to remove the wrinkles.

In the following the different aspects of the invention will be described more in detail with reference to different embodiments thereof shown, solely by way of example, in the drawings, where:
Figs. 1- 5 are diagrammatic perspective views of five different wafers. Figures 4 and 5 show embodiments of a wafer according to the invention,
Fig. 6 is a diagrammatic cross-sectional view taken along line A-A in Fig. 1 and shown in enlarged scale,
Fig. 7 is a schematic illustration of a system for scanning the peristomal skin surface and stoma of a patient and illustration of utilization of the scanning result to provide a mould,
Fig. 8 is a schematic view illustrating the method according to the invention of producing a wafer according to the invention,
Fig. 9 is a diagrammatic, broken away, partially sectional view in enlarged scale of a mould for use in the method illustrated in Fig. 8,
Figs. 10 and 11 are diagrammatic bottom plan views of two embodiments of the top mould shown in Fig. 9,
Fig. 12 is a diagrammatic, broken away, enlarged scale plan view of the spring loaded mould ring in Fig. 9,
Fig. 13 is a sectional view taken along line B-B in Fig. 12,
Fig. 14 is a diagrammatic enlarged scale view of an alternative currently preferred embodiment of a laminating station to be used for laminating a wafer according to the invention,
Figs. 15-16 are diagrammatic illustrations of the currently preferred edge configuration of the wafer according to the invention,
Figs. 17-18 are views similar to Figs. 15-16 of an alternative edge configuration not to be recommended and shown to illustrate the advantages of the preferred edge configuration of Figs. 15-16,
Figs. 19-20 are diagrammatic top views of alternative wafer configurations and
Fig. 21 is a diagrammatic cross sectional view taken along line C-C in Fig. 20.

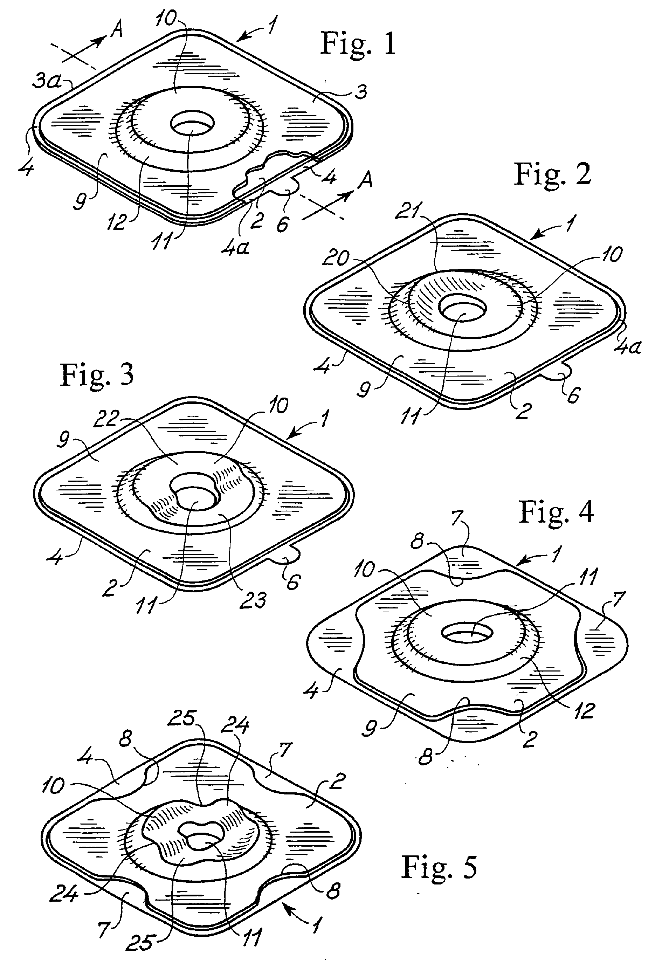

Referring now to Figs. 1 - 6, a wafer for adhesively attaching an ostomy appliance to the peristomal skin surface surrounding the stoma of a wearer of the appliance is indicated generally by the reference numeral 1. The wafer 1 comprises an adhesive layer 2 of mouldable skin barrier material sandwiched between a release sheet 3 and a flexible backing layer 4 for attaching said wafer 1 to a not shown collecting bag. The skin barrier material referred to herein may be any of the many skin barrier materials known in the art which is suitable for moulding to form the adhesive layer of a wafer according to the present invention.

In Fig. 1 the release sheet 3 has been partially cut away to show the location of the adhesive layer 2 between the release sheet 3 and the backing layer 4. In Figs. 2 - 5 the release sheet has been removed for the sake of clarity.

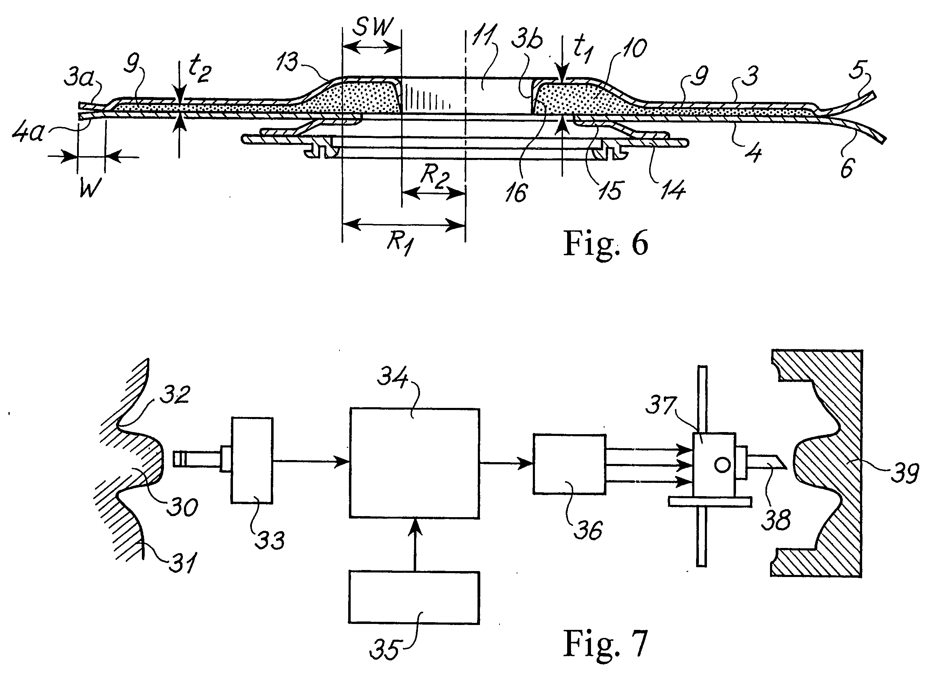

In the examples of Figs. 1-3 both the release sheet 3 and the backing layer 4 are provided with a gripping tab 5 and 6, respectively, see Fig. 6. The gripping tab 5 of the release sheet is intended for use when removing the release sheet 3 from the wafer 1, the release sheet 3 being provided with a coating of silicone or other release agent, such that the release sheet 3 can be removed prior to applying the thus exposed surface of the adhesive layer 2 of the wafer 1 to the peristomal skin surface of said wearer.

The tab 6 of the backing layer 4 is intended for being gripped by the wearer or a health care person when the wafer is to be removed from said adhesion to the peristomal skin surface by peeling the wafer off starting at the region of the adhesive layer 2 adjacent the tab 6. In the embodiments shown in Figs. 4 and 5, the tab 6 of the backing layer 4 has been substituted by areas 7 of the backing layer 4 obtained by configuring the periphery of the adhesive layer 2 with indentations or outwardly concave portions 8 such that an area 7 can be gripped for removing the wafer 1. In this manner, relatively large gripping portions 7 are obtained allowing peeling off of the wafer from said peristomal skin surface from various directions which allows greater flexibility in the operation of removing the wafer 1 from said peristomal skin surface.

Portions of the release sheet 3 corresponding to and overlying the portions 7 of the backing layer are intended to serve as gripping portions for removing the release sheet 3 from the adhesive layer 2 prior to applying the wafer 1 to said peristomal skin surface.

The indentations 8 of the periphery of the adhesive layer 8 may have other shapes, particularly in connection with other shapes of the periphery of the release sheet and backing layer, be it triangular, circular or some other shape.

Referring now to Figs. 1 and 6, the adhesive layer 2 comprises a relatively thin peripheral portion 9 and a relatively thick central portion 10. The central portion 10 is annular and concentric with a substantially circular stoma receiving aperture 11. A tapered portion 12 separates the peripheral portion 9 from the central portion 10.

The central portion 10 has a substantially uniform thickness t1 which is at least 2.5 mm larger than the thickness t2 of the peripheral portion. The substantially planar annular proximal or bodyside surface13 of the central portion has a width SW, the so-called support width.

An annular coupling ring 14 for coupling the wafer 1 to a not shown collecting bag is attached to the wafer 1 by means of an annular film 15 adhered or sealed to the ring 14 and the backing layer 4. The collecting bag may be attached in other ways to the wafer 1, either removably or non-removably, i.e. so-called two-piece and one-piece ostomy appliances.

Annular edge portions 3a and 4a of the release sheet 3 and backing layer 4, respectively, protrude beyond the periphery of the adhesive layer and have a width W between approximately 2 mm and 5 mm. The edge portion 4a of the backing layer 4 is useful in preventing any flow or leakage of adhesive from the periphery of the adhesive layer 2 from adhering to or soiling the clothes or bed clothes of the wearer.

The release sheet 3 has an inner edge portion 3b covering substantially the entire inner adhesive surface 16 of the stoma receiving aperture 11 such that the adhesive adjacent the surface 16 is protected against drying out and hardening. This is important as the surface 16 is intended to sealingly and supportingly surround and contact the not shown protruding stoma of the wearer as explained below.

A number of different wafers constituting a set of wafers are provided for selecting a specific wafer of said set most suited for matching the surface topography of the peristomal skin surface and stoma of a particular wearer of an ostomy appliance comprising said specific wafer. The criteria for selecting said specific wafer are a combination of degree of fit of the annular central portion 10 to a moat or groove in the tissue surrounding the stoma both as regards depth and width thereof, the diameter of the stoma and the necessary degree of lateral support for said stoma.

The wafers in one set of such wafers are therefore differentiated by varying the radius R1 of the annular central portion 10 and the radius R2 of the stoma aperture 11 (and consequently the support width SW) and the thickness t1 of the central portion 10. The thickness t2 of the peripheral portion is preferably constant for all wafers of the set.

### Example

In the currently preferred set of wafers of the type shown in Figs. 1 and 6, the thickness t2 is 0.85 mm. The thickness t2 may be larger or smaller if necessary.

The currently preferred set of wafers of the type shown in Figs. 1 and 6 are as specified below:

| No. | Wafer size (mm x mm) | R1 (mm) | t2 (mm) | R2 (mm) | SW (mm) |
|---|---|---|---|---|---|
| 1 | 100 x 100 | 22.0 | 3.85 | 12.5 | 9.5 |
| 2 | 100 x 100 | 18.5 | 5.85 | 9.0 | 9.5 |
| 3 | 100 x 100 | 18.5 | 5.85 | 11.5 | 8.0 |
| 4 | 100 x 100 | 22.0 | 5.85 | 12.5 | 9.5 |
| 5 | 100 x 100 | 22.0 | 5.85 | 14.0 | 8.0 |
| 6 | 115 x 115 | 25.0 | 3.85 | 16.0 | 9.0 |
| 7 | 115 x 115 | 25.0 | 3.85 | 17.5 | 7.5 |
| 8 | 115 x 115 | 25.0 | 5.85 | 16.0 | 9.0 |
| 9 | 115 x 115 | 25.0 | 5.85 | 17.5 | 7.5 |
| 10 | 115 x 115 | 27.5 | 5.85 | 19.0 | 8.5 |

The wafer size indicated above is in reality the size of the adhesive layer 2, while the sizes of the wafers 1 including the borders 3a and 4a with a width W of 3 mm are 6 mm larger on each side, i.e. 106 m x 106 mm and 121 mm x 121 mm, respectively.

The set may also comprise wafers with stoma receiving apertures having a radius R2 of 7.5 mm, these wafers being intended for use by enlarging the stoma receiving aperture by cutting with scissors or the like so as to accommodate wearers with stoma diameters and/or off-center locations that are not entirely accommodated by any of the other wafers of the set.

Furthermore, the set may comprise wafers with elliptical or oval stoma receiving apertures.

It is important to note that the radial width of the bodyside surface 13 or SW should have a minimum size of approximately 6 mm and preferably no smaller than 7 mm and most preferably no smaller than 7.5 mm. If SW is smaller, then the central portion 10 will not support a stoma inserted in the aperture 11 sufficiently in the lateral direction.

SW should not exceed 11 mm, preferably not be larger than 10 mm and most preferably not larger than 9.5 mm because for most configurations of a moat or groove around the stoma of ostomy patients having such a moat, the annular central portion 10 will not fit into the moat and the wafer will not afford correct support for a stoma inserted in the aperture 11 and will not fit well enough to adhere and seal against leakage of stomal fluids into contact with the peristomal skin surface and stoma of the wearer.

Referring now to Figs. 2-5, further examples of the wafer are illustrated. The configuration of these wafers are directed towards accomodating more differentiated surface topographies of the peristomal surface and stoma of a specific wearer.

In the example illustrated in Fig. 2, the thickness of the central portion 10 varies in both the radial and circumferential direction such that a ridge 20 with a peak 21 thereof is formed corresponding to a stoma surrounding moat with a depression having a matching topography.

In the example illustrated in Fig. 3, the thickness of the central annular portion 10 varies circumferentially such that approximately one half 22 of the central portion 10 is substantially thicker than the other half 23, for instance 6 mm and 4 mm, respectively.

In the embodiment illustrated in Fig. 4, the thickness of the central annular portion 10 is uniform as in the embodiment of Fig. 1, but the aperture 11 is oval or elliptical instead of circular so as to accommodate a stoma having an off-center location and/or a non cicular cross-section.

In the embodiment illustrated in Fig. 5, the thickness of the central portion varies both in the radial and circumferential direction so that various peaks 24 and throughs 25 are formed in the surface topography of said central portion 10 corresponding to troughs and peaks of the surface topography of the peristomal surface of a specific wearer, said peristomal surface topography being registered and reproduced in inverse relationship as explained in the following with reference to Fig. 7.

The central portion 10 may be configured with an annular periphery as illustrated in Figs. 1-5, but may also be configured with an irregular periphery corresponding to an irregular periphery of a moat surrounding a stoma of a specific wearer.

All the above described configurations may be included in a set of wafers from which a specific wearer selects the wafer best suited for the special circumstances of said specific wearer. In this manner a specific wearer may identify a standard product included in said set of wafers which will function best. However the wafer may also be customized to fit the specific wearer based on the specific surface topography of the peristomal surface and stoma of said specific wearer as described in the following with reference to Fig. 7.

In Fig. 7, a stoma 30 and a peristomal surface 31 with an exaggerated moat or groove 32 surrounding the stoma are illustrated in cross section.

A scanning mechanism 33 is arranged for scanning the topography of the stoma 30, the surface 31 and the moat 32, the scanning data being transferred to a computer 34 where a digital representation of the topography of the stoma 30 and surrounding skin surface 31 and 32 is created.

A control input device 35 causes the digital representation to be transferred to a digitally controlled activator 36 for causing a shaping device 37 with a cutting tool 38 to shape a die 39 for producing a mould for moulding the proximal bodyside surface of a wafer having a substantially perfect fit with the stoma 30 and surrounding skin surface 31, 32.

The process and equipment is illustrated in Fig. 7 in a symbolic manner and represents various possible manners of scanning the peristomal skin surface and stoma, either directly from the wearer or from a cast or impression made by means of wax or other suitable materials as well as various possible manners of providing a mould from said scanning procedure.

The scanning procedure may take place for instance either by means of laser light or ultrasound applied directly to the wearer or by x-rays applied to a cast or impression. Various different procedures to achieve such scanning and conversion into a digital representation and a die or mould are known, inter alia from US patents Nos. 4,611,288, 4,663,720, 5,056,204 and 5,487,012.

The mould for such customized moulding of the proximal surface of the wafer 1 may also be produced in a conventional manner directly from a cast or impression of the peristomal surface and stoma of a specific wearer.

The distal surface of the wafer may be provided with visual or tactile markings or guide lines that indicate the correct orientation of the wafer when being applied to the peristomal surface such that the fit between the proximal surface of the wafer and said peristomal surface is obtained.

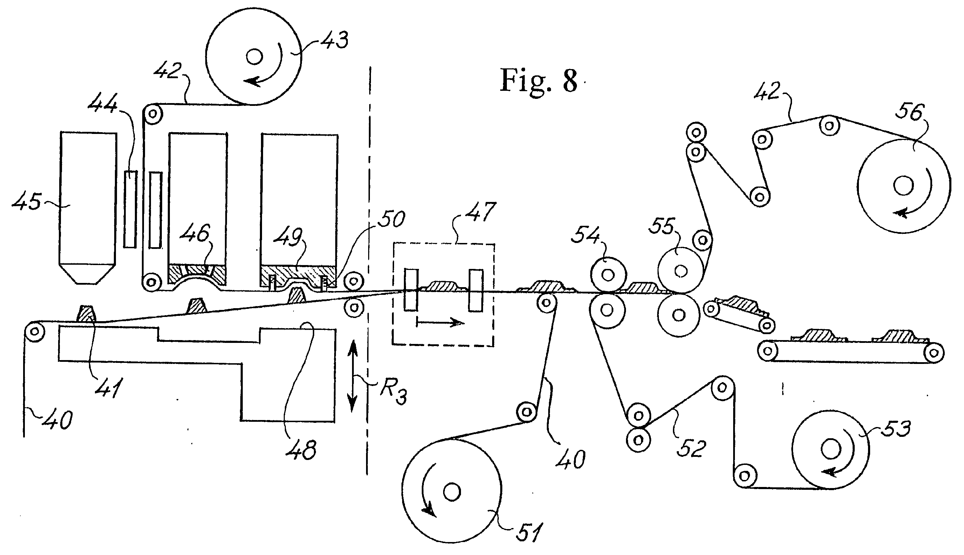

Referring now to Fig. 8, a sacrificial or support web 40 of PET coated with a release agent is supplied from a not shown roll, and patties 41 of a skin barrier material are deposited thereon from a container 45 thereof. A release web 42 with a thickness of approximately 0.15 mm and coated with a release agent such as silicone is supplied from a roll 43 thereof and preheated to approximately 120 degrees centigrade at a heating station 44 and thereafter pre-formed in a vacuum mould, the web being sucked unto the mould surface by vacuum supplied to channels 46 such that a pre-formed area of the release liner 42 is obtained.

An indexing station 47 moves the sacrificial web 40 and the release web forward step-wise such that a precise register of webs 40 and 42 takes place relative to one another and the various treatment stations in the process train.

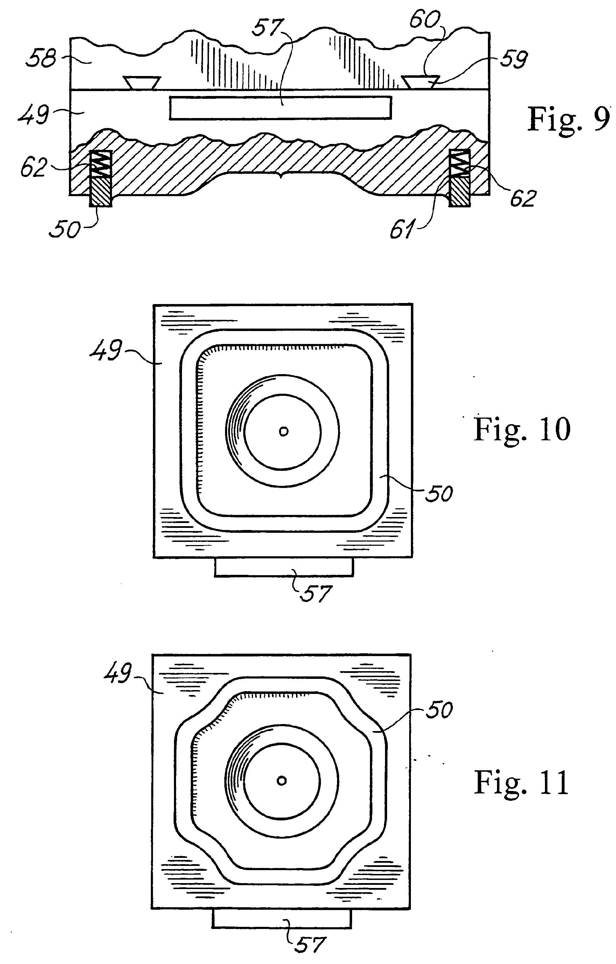

The pre-formed area of the release web 42 is located centrally over a patty 41 between a lower mould part 48 and an upper mould part 49 provided with a spring loaded annular body 50 described more in detail in the following with reference to Figs. 9-13. The upper mould 49 has a moulding surface very similar to and preferably identical with the moulding surface of the vacuum mould 46.

The lower mould 48 is pressed upwards as indicated by the arrow R3 so that the patty 41 and the preformed area of the release web 42 are moulded to the desired topography of the proximal surface of the final wafer 1. As the release web 42 has been preformed to the desired surface topography only the patty material is to be moulded by the pressure exerted by the movable mould part 48.

After the indexing station 47 the support web 40 is peeled away from the moulded patties and wound up on a roll 51, and a corona treated EMA backing web 52 is supplied from a roll 53 thereof and laminated to the distal surface of the adhesive layer (patty) at a laminating station 54. Finally, the finished wafers 1 are cut out at a rolling cutting station cutting through the release web 42 and the backing web 52, the cutting waste of the webs 42 and 52 being wound up on a waste roller 56. The wafers 1 are transported to a not shown punching station where the stoma receiving aperture 11 of the wafer 1 is formed.

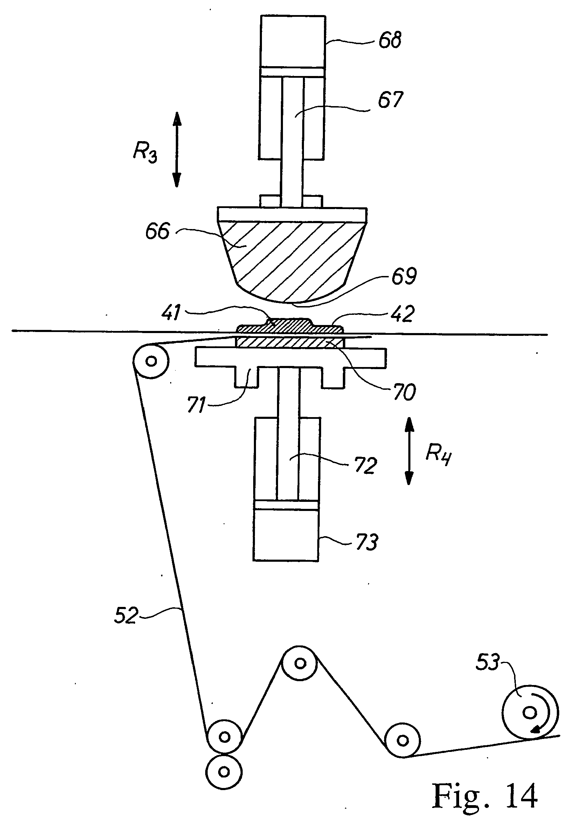

The laminating station 54 comprises two laminating rollers having a resilient surface configuration and being pressed against each other so as to apply a laminating pressure to the release web 42 and the backing web 52 such that these webs are laminated to the layer of adhesive skin barrier material 41 when passing between the two laminating rollers. In the following an alternative and currently preferred laminating station will be described in connection with Fig. 14.

Referring now to Figs. 9-11, the upper mould 49 in Fig. 8 is provided with a handle 57 and is attached to a supporting body 58 by means of two ribs 59 displaceably inserted in corresponding grooves 60 in the supporting body 58 such that the mould 49 can be substituted quickly by another mould. This attachment system is also utilized for the vacuum mould 46 so that this mould also may be replaced quickly.

The annular body 50 is received displaceably in an annular groove 61 and may be displaced upwards in said groove against the force of compression springs 62. Not shown cooperating shoulders on the body 50 and the inner surface of the groove 61 prevent the body 50 from being pressed entirely out of the groove by the springs 62.

When a wafer 1 is being moulded in the mould 48, 49, the body 50 functions as a stop ring to prevent barrier material of the patty 41 from being pressed beyond the stop ring 50 that is kept pressed against the release web 42 and the lower mould 48 by the springs 62 during the moulding operation whereby a sharply defined periphery of the adhesive layer of the wafer is formed and no adhesive is pressed onto the ring 4a of backing material (see Fig. 6).

In Fig. 10, stop ring 50 corresponding to the periphery of the adhesive layer 2 in Figs. 1-3 is shown, while the stop ring 50 shown in Fig. 11 corresponds to the periphery shown in Fig. 4.

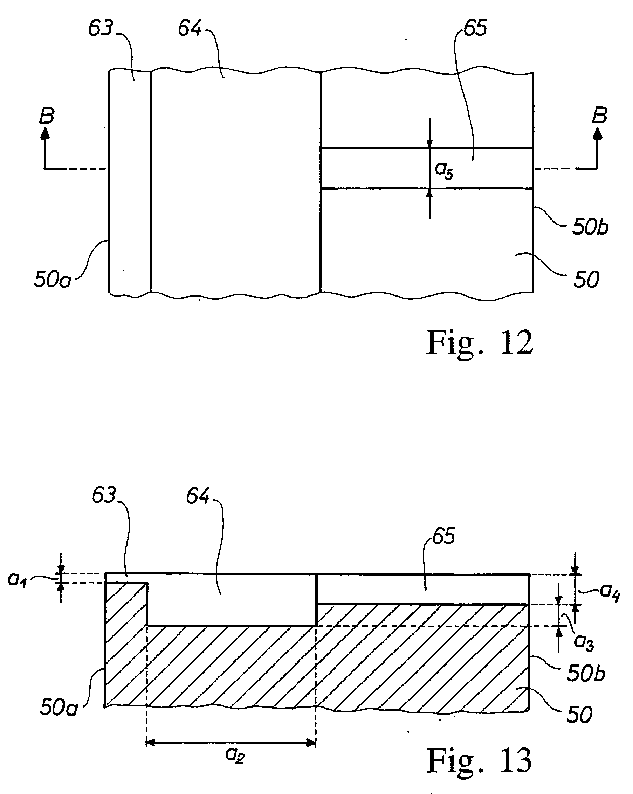

Referring now to Figs. 12-13, the surface of the stop ring opposite the springs 62 is provided with an annular ventilating or air bleeding gap 63 adjacent the inner surface 50a of the stop ring 50 for allowing any air pressed out to the periphery of the wafer by the moulding process to escape into an annular groove 64 communicating with the surrounding air through regularly spaced radial grooves 65, the indicated dimensions a1 to a5 being a1 = 0.1 mm, a2 = 4 mm, a3 = 1 mm, a4 =3 mm and a5 =5 mm. Hereby it is avoided that any air entrapped between the moulds and the wafer or between the webs 42 and 40 does not give rise to irregularities in the adhesive wafer. The viscosity of the mouldable skin barrier material is such that substantially no skin barrier material is pressed into the air bleeding gap 63.

The air bleeding passage 63, 64, 65 may be configured in many other ways obvious to the person skilled in the art, for instance by providing regularly spaced radial ribs having a height of approx. 0.1 mm and each extending between the inner surface 50a to the outer surface 50b of the stop ring 50.

Referring now to Fig. 14, an alternative and currently preferred laminating station for use instead of the laminating station 54 in Fig. 8 comprises a resilient lamination pad 66 mounted on a piston 67 displaceable up and down in the direction R3 in a pneumatic cylinder 68 such that the pad 66 may exert a laminating pressure on the wafer 41 and the webs 42 and 52 for laminating them together.

The surface portion 69 of the pad 66 that contacts the wafer 41 during the lamination process is dome shaped so that the laminating pressure is applied starting at the centre of the wafer 41 and moving outwards towards the periphery of the wafer 41. Hereby any air entrapped between the webs 42 and 48 will be pressed out and the stresses in the web 52 will be relaxed such that deformation of the wafer 41 by remaining stresses in the release sheet 52 is limited or eliminated.

A resilient counter pressure pad 70 is arranged on a table 70 attached to a piston 72 displaceable up and down in the direction R4 in a pneumatic cylinder 73 such that the counter pressure pad 70 may exert a laminating counter pressure on the wafer 41 and move downwards to allow the web 52 to be released from contact with the pad 70 after the lamination stroke upwards of the pad 70.

The lamination pad 66 is a printer's pad or tampon designed for applying printing to a surface and is made of silicone with a hardness SHORE A6 while the counter pressure pad 70 is either of neoprene or silicone with a hardness SHORE A15. The lamination pad or tampon used with good results was supplied by the company Morlock Tampondruck Systeme GmbH, Lise-Meitner-Str. 9, Dornstetten, Germany.

The maximum pressure applied by the lamination pad 66 to the surface of the wafer 41 is approximately 1900 N/cm2.

The counter pressure pad 70 may have a plane surface facing the pad 66 or this surface may be upwardly domed or convex to reinforce the effect discussed above of the laminating pressure being applied from the centre towards the periphery of the wafer 41.

The pads 66 and 70 may be made of many other resilient materials such as rubber, latex, plastic foam material and so on, and they may be solid or hollow. The counter pressure pad 70 is not absolutely necessary.

The hardness of the pads 66 and 70 may vary according to the type of skin barrier material used in the wafer 41.

The lamination process by means of the pads 66 and 70 results in much fewer defectively laminated wafers than the lamination process by means of rollers shown in Fig. 8.

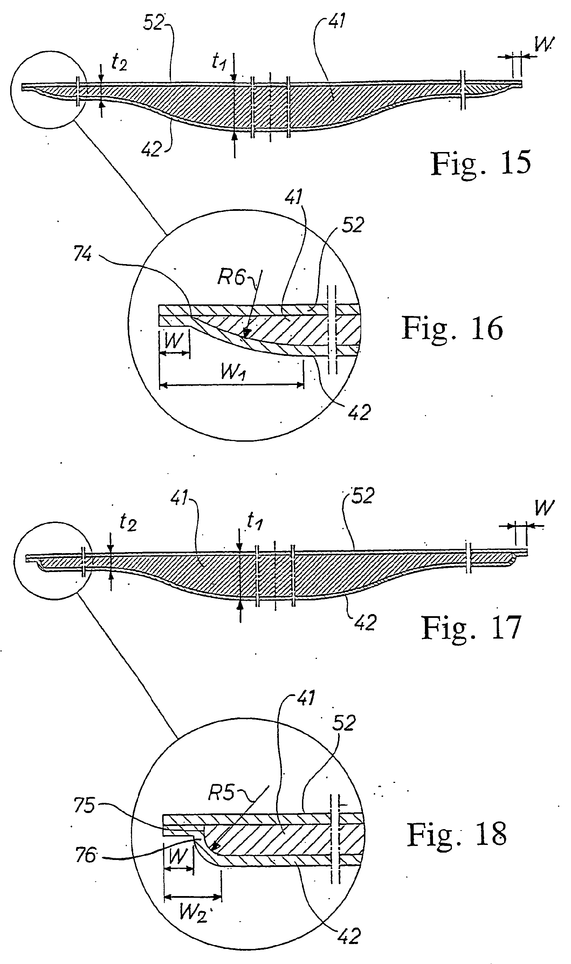

The results are particularly good when the lamination process of Fig. 14 is utilised in connection with the currently preferred embodiment of the wafer according to the invention having the edge or periphery configuration according to the invention shown in Figs. 15-16.

Referring now to Figs. 15-18, the currently preferred configuration of the periphery of the wafer 41 is illustrated in Fig. 15-16 as compared to a not recommendable edge configuration illustrated in Figs. 17-18.

The edge 74 of the wafer 41 in Figs 15-16 is sharp as it tapers gradually to a very small thickness as compared to the edge 75 of the wafer 42 of Figs. 17-18 that has an appreciable thickness and is blunt giving rise to a substantially vertical peripheral region 76 of the release sheet 42 extending at substantially right angles to the web 52 where the comparable peripheral region of the release sheet in Fig. 16 forms a relatively small acute angle with the web 52.

The curvature radius R6 in Fig. 16 is 45 mm and W1 is 10 mm while the curvature radius R5 in Fig. 18 is 1.5 mm and W2 is 4.2 mm with W = 3 mm in both Figures.

As mentioned above, the laminating results in connection with the embodiment of Figs. 15-16, particularly when utilising the tampon of Fig. 14 are much better than with the embodiment of Figs. 17-18 as the edge portion adjacent the edge 74 of the wafer 41 adheres much better to the carrier web 52 and the release web 42.

One of the beneficial results of this better adherence is that the edge portion adjacent the edge 74 is much less prone to drying out than the edge portion adjacent the edge 75. The edge of the wafer 41 of Figs. 15-16 is isolated from contact with the surrounding atmosphere by the good adherence to the webs 42 and 52 and the only portion of the edge in contact with the atmosphere is the sharp edge 74.

To the contrary, in the wafer edge of Figs. 17-18 the adhesive layer 41 does not adhere well to the release liner 42 along the vertical portion 76 thereof, and furthermore the adhesive layer 41 does not adhere well to the carrier web 52 along an annular strip extending along and adjacent to the edge 75. Thus, the surrounding atmosphere is in drying out contact with a relatively large surface of the edge of the wafer giving rise to detrimental drying out of a relatively wide peripheral ring of the edge of the wafer in Figs. 17-18.

It is currently believed that the major reason for achievement of the beneficial effect is as explained in the following.

The laminating pressure from the laminating rollers in Fig. 8 and particularly from the laminating pad 66 in Fig. 14 is applied in a vertical direction substantially perpendicular to the plane of the carrier web 52 and substantially parallel to the vertical portion 76 of the release web 42. Hereby no substantial laminating pressure is applied to the end surface 75 of the adhesive wafer and the vertical portion 76 of the release sheet and therefore no substantial lamination takes place here.

Furthermore, the vertical portion 76 of the relatively thick and stiff release web 42 will transmit the laminating pressure at the edge of the wafer 41 directly to the carrier web 52 as a linear force and not through the material of the wafer whereby the carrier web 52 will be pressed away from the adhesive layer instead of being laminated thereto which causes the bad adhesion between the carrier web and the wafer 41 adjacent the edge surface 75.

By ensuring that the laminating pressure in the embodiment of Figs. 15-16 is transmitted to the wafer 41 in a direction considerably transverse to the release sheet 42 at all points of the edge region thereof, good laminating pressure is ensured between the entire surface of the release sheet 42 and the wafer as well as between the entire surface of the carrier web 52 and the wafer thus ensuring good adhesion between the wafer and both webs along the entire edge portion of the wafer 41.

This is ensured by arranging the entire edge region surface of the release sheet at an angle to the direction of the laminating pressure (substantially vertical in Fig. 16) of between 45 and 90 degrees, preferably between 70 and 90 degrees. In other words, the angle between the release sheet 42 and the carrier 52 at the edge region should be between 0 and 45 degrees, preferably between 0 and 20 degrees.

The thickness t2 will typically be between 0.8 and 1.2 mm, and one of the objectives of configuring the outer peripheral edge region of the adhesive layer with a gradually tapering thickness is to achieve as small a thickness of the layer at the edge 74 as possible. In practice, because of the particle size of the adhesive material and the viscosity thereof, the thickness of the adhesive layer 41 at the edge 74 will be between 0.1 mm and 0.3 mm.

The tapering effect may be achieved by means of a rectilinear radial profile of the release sheet 42 at the edge region instead of the circular radial profile shown in Fig. 16.

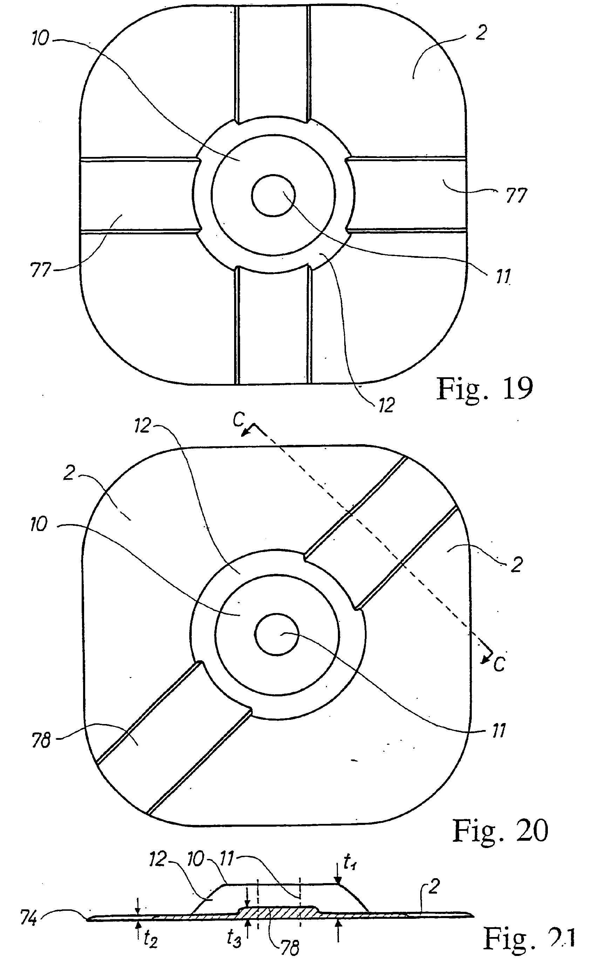

Referring now to Figs 19-21, alternative examples of the wafer are shown with radially extending thickened portions 77 and 78 (Figs. 19 and 20-21, respectively) extending to the peripheral edge of the peripheral portion 2. The thickness t3 is approximately one third of the thickness t1 of the central portion 10, for instance t1 = 5.0 mm, t2 = 0.8 mm and t3 = 1.7 mm.

The thickness t3 is preferably constant along the entire extent of the thickened portions 77 and 78 but may also vary to comply with special patient requirements.

The object of the thickened radially extending portions is to provide regions with better adhesive properties because of the thicker layer of adhesive. Furthermore, the wafer may be oriented such that the thickened portions register with natural folds in the peristomal tissue, either permanent folds or folds occurring when sitting down. The wafer in Fig. 20 may be oriented such that the thickened portion is substantially horizontal so as to fit into a crease forming in the peristomal tissue when sitting or bending down.

While several embodiments of the different aspects of the invention have been described, other embodiments are conceivable without departing from the scope of the invention as defined by the appended patent claims.

## Claims

1. A wafer (1) for adhesively attaching an ostomy appliance to a peristomal skin surface surrounding a stoma of a wearer of said appliance, said wafer comprising an integrally moulded adhesive layer (2) consisting of a moisture absorbing and swellable skin barrier material and having a substantially planar distal surface covered by a flexible backing layer (4), extending at least to the outer periphery of the adhesive layer (2), the shape of said outer periphery consisting of outwardly convex or substantially rectilinear portions, for attaching said wafer to a collecting bag, and a proximal bodyside surface that is contoured such that said adhesive layer (2) has a relatively thin peripheral portion (9) and a relatively thick central portion (10) surrounding an aperture for receiving said stoma, said central portion (10) being distributed substantially uniformly around said aperture **characterised by** said central portion (10) having a maximum thickness which is at least 2.5 mm larger than the maximum thickness of said peripheral portion, wherein the backing layer (4) includes a gripping tab or a gripping portion (7) defined by a portion of the backing layer that is free of the adhesive layer (2), which is provided by the adhesive layer (2) having an indentation (8) in the outer periphery being arranged on the backing layer (4) free of the indentation.

2. A wafer according to claim 1, wherein said peripheral portion has a width of at least 10 mm, preferably at least 12 mm and most preferably at least 15 mm.

3. A wafer according to claim 1 or 2, wherein the thickness of said peripheral portion is at least 0.4 mm, preferably at least 0,5 mm, more preferably at least 0.6 mm, even more preferably at least 0.7 mm and most preferably at least 0.8 mm.

4. A wafer according to any of the preceding claims, wherein said central portion is annular.

5. A wafer according to claim 4, wherein said aperture is substantially circular and concentric with said annular central portion.

6. A wafer according to any of the claims 1-5, wherein said central portion has a radial width of at least 6 mm, preferably at least 7 mm and most preferably larger than or equal to 7.5 mm.

7. A wafer according to any of the claims 1-6, wherein said central portion has a radial width of less than 11 mm, preferably less than 10 mm, most preferably less than or equal to 9.5 mm.

8. A wafer according to any of the preceding claims, wherein said central portion has a substantially uniform thickness.

9. A wafer according to any of the claims 1-7, wherein said central portion has a thickness that varies in the circumferential direction.

10. A wafer according to any of the claims 1-7 or 9, wherein said central portion has a thickness that varies in the radial direction.

11. A wafer according to any of the preceding claims, wherein said proximal surface is covered by a flexible release sheet removably attached to said proximal surface, said release sheet extending at least to the outer periphery of said adhesive layer.

12. A wafer according to claim 11, wherein an edge region of said adhesive layer being at least 3 mm wide, preferably at least 6 mm wide and extending along and adjacent to the entire length of said outer periphery is configured such that the angle between all mutually opposing portions of said release sheet and said flexible backing layer covering said edge region is less than 45 degrees, preferably less than 35 degrees, more preferably less than 30 degrees, even more preferably less than 25 and most preferably less than 20 degrees.

13. A wafer according to claim 12, wherein the thickness of said adhesive layer at said outer periphery is less than 0.5 mm, preferably less than 0.4 mm and most preferably less than 0.3 mm.

14. A wafer according to claim 12, wherein an edge region of said adhesive layer being at least 3 mm wide, preferably at least 6 mm wide and extending along and adjacent to the entire length of said outer periphery is configured such that said proximal and distal surfaces in said edge region taper towards each other in the direction towards said outer periphery, and the thickness of said adhesive layer at said outer periphery is less than 0.5 mm, preferably less than 0.4 mm and most preferably less than 0.3 mm.

15. A wafer according to any of the preceding claims, wherein a thickened region of said relatively thin peripheral portion of said adhesive layer has a thickness substantially larger than the maximum thickness of the rest of said peripheral portion.

16. A wafer according to claim 15, wherein said thickened region extends between said relatively thick central portion and the outer periphery of said adhesive layer.

17. A wafer according to claim 15 or 16, wherein the thickness of said thickened region is uniform and is between 30% and 50% of the maximum thickness of said central portion.

18. A set of at least two wafers for adhesively attaching an ostomy appliance to a peristomal skin surface surrounding a stoma of a wearer of said appliance, each wafer of said set is according to claims 1-17 and comprises an adhesive layer consisting of a moisture absorbing and swellable skin barrier material and having a substantially planar distal surface covered by a flexible backing layer, extending at least to the outer periphery of the adhesive layer, for attaching said wafers to a collecting bag and a proximal bodyside surface that is contoured such that said adhesive layer has a relatively thin peripheral portion and a relatively thick central portion surrounding an aperture for receiving said stoma, said central portion being distributed substantially uniformly around said aperture and having a maximum thickness which is at least 2.5 mm larger than the maximum thickness of said peripheral portion, the individual wafers of said set differing from one another in that the topography of the surface of said central portion is different.

19. A set of wafers according to claim 18, wherein at least two of the wafers of the set is in accordance with any of the claims 2-17.

20. A set of wafers according to claim 18 or 19, wherein at least two wafers differ from one another in that the size and/or shape of said stoma receiving aperture is different.

21. A method of producing a wafer for adhesively attaching an ostomy appliance to a peristomal skin surface surrounding a stoma of a wearer of said appliance, said wafer comprising an integrally moulded adhesive layer consisting of a moisture absorbing and swellable skin barrier material and having a substantially planar distal surface covered by a flexible backing layer, extending at least to the outer periphery of the adhesive layer, the shape of said outer periphery consisting of outwardly convex or substantially rectilinear portions, for attaching said wafer to a collecting bag and a proximal contoured bodyside surface having a first surface topography such that said adhesive layer has a relatively thin peripheral portion and a relatively thick central portion surrounding an aperture for receiving said stoma, said proximal surface being covered by a release layer and said central portion having a maximum thickness which is at least 2.5 mm larger than the maximum thickness of said peripheral portion, wherein the backing layer (4) includes a gripping tab (6) or a gripping portion (7) defined by a portion of the backing layer that is free of the adhesive layer (2), which is provided by the adhesive layer (2) having an indentation (8) in the outer periphery being arranged on the backing layer (4) free of the indentation, the method comprising the steps of: - providing a first mould for pre-forming said release layer and provided with a first mould hollow having a second surface topography similar to, preferably substantially identical to said first surface topography, said hollow being provided with suction apertures communicating with a vacuum source, providing a second mould for moulding said adhesive layer having a first part and a second part adapted for being pressed against one another, said first part being provided with a second mould hollow for defining said first surface topography of said adhesive layer, providing a substantially continuous supply of a sacrificial web, - arranging said skin barrier material on said sacrificial web, providing a substantially continuous supply of release web, - heating an area of said release web for enhancing the deformability thereof, -locating said heated area in register with said first mould hollow, applying vacuum to said suction apertures such that said area is sucked into said hollow and thereby deformed such that said area is provided with a third surface topography substantially identical to said second surface topography, - relieving said vacuum application, - displacing said area into register with said second mould hollow such that said third surface topography with said second surface topography, -displacing a portion of said sacrificial web with barrier material thereon into register with said second mould hollow such that said area is located between said portion and said second mould hollow, and pressing said second part against said first part such that said area and said portion are pressed into said second mould hollow.

22. A method of laminating a wafer according to any of the claims 1-17, wherein said method comprises the following steps: - applying said flexible backing layer to said distal surface, - applying a flexible release sheet to said proximal surface, - providing a resilient laminating pad or tampon adapted for being displaced to and fro in a laminating direction and for exerting a laminating pressure in said laminating direction, - providing a plane counter-pressure surface at substantially right angles to said laminating direction, - placing said wafer on said counter-pressure surface with said plane backing layer abutting said counter-pressure surface, and - displacing said laminating pad in said laminating direction such that laminating pressure is exerted on the entire distal surface of said wafer by said laminating pad.

23. A method according to claim 22, wherein the surface of said laminating pad destined to exert said laminating pressure on said wafer is domed or convex in said laminating direction.

24. A method according to claim 22 or 23, wherein said laminating pad is made of silicone.

25. A method according to any of the claims 22-24, wherein said laminating pad is a printer's pad designed for printing on a surface.

26. A method according to any of the claims 22-25, wherein said laminating pad has a hardness between SHORE A3 and SHORE A9, preferably between SHORE A5 and SHORE A7.

27. A method according to any of the claims 22-26, wherein said counter-pressure surface is adapted for being displaced to and fro in said laminating direction.

28. A method according to any of the claims 22-27, wherein said counter-pressure surface is the surface of a resilient counter-pressure pad.

29. A method according to claim 28, wherein said counter-pressure pad is made of silicone or neoprene.

30. A method according to claim 28 or 29, the surface of said counter-pressure pad destined to abut said backing layer is domed or convex in the direction opposite said laminating direction.

## Patentansprüche

1. Scheibe (1) zum klebenden Befestigen einer Stoma-Vorrichtung auf einer peristomalen Hautoberfläche, die ein Stoma eines Trägers der Vorrichtung umgibt, wobei die Scheibe eine integral geformte klebende Schicht (2) umfasst, die aus einem feuchtigkeitsabsorbierenden und quellfähigen Hautbarrierematerial besteht und eine im Wesentlichen ebene distale Fläche aufweist, bedeckt von einer flexiblen Rückschicht (4), die sich wenigstens bis zur äußeren Peripherie der klebenden Schicht (2) erstreckt, wobei die Form der äußeren Peripherie aus nach außen konvexen oder im Wesentlichen geradlinigen Abschnitten zum Befestigen der Scheibe an einem Sammelbeutel besteht, und eine proximale körperseitige Fläche, die so konturiert ist, dass die klebende Schicht (2) einen relativ dünnen peripheren Abschnitt (9) und einen relativ dicken mittigen Abschnitt (10) aufweist, der eine Öffnung zum Aufnehmen des Stomas umgibt, wobei der mittige Abschnitt (10) im Wesentlichen gleichmäßig um die Öffnung verteilt ist, **dadurch gekennzeichnet, dass** der mittige Abschnitt (10) eine maximale Dicke aufweist, die wenigstens 2,5 mm größer ist als die maximale Dicke des peripheren Abschnitts, wobei die Rückschicht (4) eine Greiflasche oder einen Greifabschnitt (7) umfasst, definiert durch einen Abschnitt der Rückschicht, der frei von der klebenden Schicht (2) ist, der dadurch bereitgestellt wird, dass die klebende Schicht (2) eine Vertiefung (8) in der äußeren Peripherie aufweist, die frei von der Vertiefung auf der Rückschicht (4) angeordnet ist.

2. Scheibe nach Anspruch 1, wobei der periphere Abschnitt eine Breite von wenigstens 10 mm, bevorzugt wenigstens 12 mm und am bevorzugtesten wenigstens 15 mm aufweist.

3. Scheibe nach Anspruch 1 oder 2, wobei die Dicke des peripheren Abschnitts wenigstens 0,4 mm, bevorzugt wenigstens 0,5 mm, bevorzugter wenigstens 0,6 mm, noch bevorzugter 0,7 mm und am bevorzugtesten wenigstens 0,8 mm beträgt.

4. Scheibe nach einem der vorhergehenden Ansprüche, wobei der mittige Abschnitt ringförmig ist.

5. Scheibe nach Anspruch 4, wobei die Öffnung im Wesentlichen kreisförmig und konzentrisch mit dem kreisförmigen mittigen Abschnitt ist.

6. Scheibe nach einem der Ansprüche 1-5, wobei der mittige Abschnitt eine radiale Breite von wenigstens 6 mm, bevorzugt wenigstens 7 mm und am bevorzugtesten größer oder gleich 7,5 mm aufweist.

7. Scheibe nach einem der Ansprüche 1-6, wobei der mittige Abschnitt eine radiale Breite von weniger als 11 mm, bevorzugt weniger als 10 mm, am bevorzugtesten weniger als oder gleich 9,5 mm aufweist.

8. Scheibe nach einem der vorhergehenden Ansprüche, wobei der mittige Abschnitt eine im Wesentlichen einheitliche Dicke aufweist.

9. Scheibe nach einem der Ansprüche 1-7, wobei der mittige Abschnitt eine Dicke aufweist, die in Umfangsrichtung variiert.

10. Scheibe nach einem der Ansprüche 1-7 oder 9, wobei der mittige Abschnitt eine Dicke aufweist, die in radialer Richtung variiert.

11. Scheibe nach einem der vorhergehenden Ansprüche, wobei die proximale Fläche von einer an der proximalen Fläche entfernbar befestigten flexiblen Abziehfolie bedeckt ist, wobei sich die Abziehfolie wenigstens bis zur äußeren Peripherie der klebenden Schicht erstreckt.

12. Scheibe nach Anspruch 11, wobei ein Randbereich der klebenden Schicht, der wenigstens 3 mm breit, bevorzugt wenigstens 6 mm breit ist und sich angrenzend an die äußere Peripherie über deren gesamte Länge erstreckt, so konfiguriert ist, dass der Winkel zwischen allen einander gegenüberliegenden Abschnitten der Abziehfolie und der den Randbereich bedeckenden flexiblen Rückschicht weniger als 45 Grad, bevorzugt weniger als 35 Grad, bevorzugter weniger als 30 Grad, noch bevorzugter weniger als 25 und am bevorzugtesten weniger als 20 Grad beträgt.

13. Scheibe nach Anspruch 12, wobei die Dicke der klebenden Schicht an der äußeren Peripherie weniger als 0,5 mm, bevorzugt weniger als 0,4 mm und am bevorzugtesten weniger als 0,3 mm beträgt.

14. Scheibe nach Anspruch 12, wobei ein Randbereich der klebenden Schicht, der wenigstens 3 mm breit, bevorzugt wenigstens 6 mm breit ist und sich angrenzend an die äußere Peripherie über deren gesamte Länge erstreckt, so konfiguriert ist, dass die proximale und distale Fläche in dem Randbereich in Richtung der äußeren Peripherie aufeinander zulaufen und die Dicke der klebenden Schicht an der äußeren Peripherie weniger als 0,5 mm, bevorzugt weniger als 0,4 mm und am bevorzugtesten weniger als 0,3 mm beträgt.

15. Scheibe nach einem der vorhergehenden Ansprüche, wobei ein verdickter Bereich des relativ dünnen peripheren Abschnitts der klebenden Schicht eine Dicke aufweist, die im Wesentlichen größer ist als die maximale Dicke des restlichen peripheren Abschnitts.

16. Scheibe nach Anspruch 15, wobei sich der verdickte Bereich zwischen dem relativ dicken mittleren Abschnitt und der äußeren Peripherie der klebenden Schicht erstreckt.

17. Scheibe nach Anspruch 15 oder 16, wobei die Dicke des verdickten Bereichs einheitlich ist und zwischen 30 % und 50 % der maximalen Dicke des mittigen Abschnitts beträgt.

18. Satz von wenigstens zwei Scheiben zum klebenden Befestigen einer Stoma-Vorrichtung auf einer peristomalen Hautoberfläche, die ein Stoma eines Trägers der Vorrichtung umgibt, wobei jede Scheibe des Satzes den Ansprüchen 1-17 entspricht und eine klebende Schicht umfasst, die aus einem feuchtigkeitsabsorbierenden und quellfähigen Hautbarrierematerial besteht und eine im Wesentlichen ebene distale Fläche aufweist, bedeckt von einer flexiblen Rückschicht, die sich wenigstens bis zur äußeren Peripherie der klebenden Schicht erstreckt, um die Scheiben an einem Sammelbeutel zu befestigen, und eine proximale körperseitige Fläche, die so konturiert ist, dass die klebende Schicht einen relativ dünnen peripheren Abschnitt und einen relativ dicken mittigen Abschnitt aufweist, der eine Öffnung zum Aufnehmen des Stomas umgibt, wobei der mittige Abschnitt im Wesentlichen gleichmäßig um die Öffnung verteilt ist und eine maximale Dicke aufweist, die wenigstens 2,5 mm größer ist als die maximale Dicke des peripheren Abschnitts, wobei sich die einzelnen Scheiben des Satzes dadurch voneinander unterscheiden, dass die Flächentopographie des mittigen Abschnitts unterschiedlich ist.

19. Satz von Scheiben nach Anspruch 18, wobei wenigstens zwei der Scheiben des Satzes einem der Ansprüche 2-17 entsprechen.

20. Satz von Scheiben nach Anspruch 18 oder 19, wobei sich wenigstens zwei Scheiben dadurch voneinander unterscheiden, dass die Größe und/oder Form der das Stoma aufnehmenden Öffnung unterschiedlich ist.

21. Verfahren zur Herstellung einer Scheibe zum klebenden Befestigen einer Stoma-Vorrichtung auf einer peristomalen Hautoberfläche, die ein Stoma eines Trägers der Vorrichtung umgibt, wobei die Scheibe eine integral geformte klebende Schicht umfasst, die aus einem feuchtigkeitsabsorbierenden und quellfähigen Hautbarrierematerial besteht und eine im Wesentlichen ebene distale Fläche aufweist, bedeckt von einer flexiblen Rückschicht, die sich wenigstens bis zur äußeren Peripherie der klebenden Schicht erstreckt, wobei die Form der äußeren Peripherie aus nach außen konvexen oder im Wesentlichen geradlinigen Abschnitten zum Befestigen der Scheibe an einem Sammelbeutel besteht, und eine proximale konturierte körperseitige Fläche mit einer ersten Flächentopografie, so dass die klebende Schicht einen relativ dünnen peripheren Abschnitt und einen relativ dicken mittigen Abschnitt aufweist, der eine Öffnung zum Aufnehmen des Stomas umgibt, wobei die proximale Fläche von einer Abziehschicht bedeckt ist und der mittige Abschnitt eine maximale Dicke aufweist, die wenigstens 2,5 mm größer ist als die maximale Dicke des peripheren Abschnitts, wobei die Rückschicht (4) eine Greiflasche (6) oder einen Greifabschnitt (7) umfasst, definiert durch einen Abschnitt der Rückschicht, der frei von der klebenden Schicht (2) ist, der dadurch bereitgestellt wird, dass die klebende Schicht (2) eine Vertiefung (8) in der äußeren Peripherie aufweist, die frei von der Vertiefung auf der Rückschicht (4) angeordnet ist, wobei das Verfahren die folgenden Schritte umfasst: -Bereitstellen einer mit einer ersten Formaushöhlung ausgestatteten ersten Form zum Vorformen der Abziehschicht mit einer zweiten Flächentopographie, die ähnlich wie die oder bevorzugt im Wesentlichen identisch mit der ersten Flächentopographie ist, wobei die Aushöhlung mit Saugöffnungen ausgestattet ist, die mit einer Vakuumquelle kommunizieren, eine zweite Form bereitstellend, um die klebende Schicht mit einem ersten und einem zweiten Teil, die dafür ausgerichtet sind, gegeneinander gepresst zu werden, zu formen, wobei der erste Teil mit einer zweiten Formaushöhlung ausgestattet ist, um die erste Flächentopographie der klebenden Schicht zu definieren, eine im Wesentlichen kontinuierliche Zufuhr einer Opferschicht bereitstellend, -Anordnen des Hautbarrierematerials auf der Opferschicht, eine im Wesentlichen kontinuierliche Zufuhr der Abziehschicht bereitstellend, -Erhitzen eines Bereichs der Abziehschicht, um deren Verformbarkeit zu verbessern, -Positionieren des erhitzten Bereichs im Einklang mit der ersten Formaushöhlung, Anwenden von Vakuum auf die Saugöffnungen, so dass der Bereich in die Aushöhlung gesaugt und dabei verformt wird, so dass der Bereich mit einer dritten Flächentopographie ausgestattet ist, die im Wesentlichen mit der zweiten Flächentopographie identisch ist, -Aufheben der Vakuumanwendung, -Verschieben des Bereichs im Einklang mit der zweiten Formaushöhlung, so dass die dritte Flächentopographie mit der zweiten Flächentopographie, -Verschieben eines Abschnitts der Opferschicht mit Barrierematerial darauf im Einklang mit der zweiten Formaushöhlung, so dass sich der Bereich zwischen dem Abschnitt und der zweiten Formaushöhlung befindet, und Pressen des zweiten Teils gegen den ersten Teil, so dass der Bereich und der Abschnitt in die zweite Formaushöhlung gepresst werden.

22. Verfahren zum Laminieren einer Scheibe nach einem der Ansprüche 1-17, wobei das Verfahren die folgenden Schritte umfasst: -Aufbringen der flexiblen Rückschicht auf die distale Fläche, -Aufbringen einer flexiblen Abziehfolie auf die proximale Fläche, -Bereitstellen eines elastischen Laminierungspolsters oder -tampons, das oder der dafür ausgelegt ist, in eine Laminierungsrichtung vor und zurück verschoben zu werden und in Laminierungsrichtung Laminierungsdruck auszuüben, -Bereitstellen einer ebenen Gegendruckfläche mit im Wesentlichen rechten Winkeln zur Laminierungsrichtung, - Platzieren der Scheibe auf der Gegendruckfläche, wobei die ebene Rückschicht an die Gegendruckfläche anstößt, und -Verschieben des Laminierungspolsters in Laminierungsrichtung, so dass von dem Laminierungspolster Laminierungsdruck auf die gesamte distale Fläche der Scheibe ausgeübt wird.

23. Verfahren nach Anspruch 22, wobei die Fläche des zum Ausüben des Laminierungsdrucks auf die Scheibe bestimmten Laminierungspolsters in Laminierungsrichtung gewölbt oder konvex ist.

24. Verfahren nach Anspruch 22 oder 23, wobei das Laminierungspolster aus Silikon besteht.

25. Verfahren nach einem der Ansprüche 22-24, wobei das Laminierungspolster ein zum Drucken auf eine Fläche entwickeltes Druckpolster ist.

26. Verfahren nach einem der Ansprüche 22-25, wobei das Laminierungspolster eine Härte zwischen SHORE A3 und SHORE A9, bevorzugt zwischen SHORE A5 und SHORE A7 hat.

27. Verfahren nach einem der Ansprüche 22-26, wobei die Gegendruckfläche angepasst ist, um in Laminierungsrichtung vor und zurück verschoben zu werden.

28. Verfahren nach einem der Ansprüche 22-27, wobei die Gegendruckfläche die Fläche eines elastischen Gegendruckpolsters ist.

29. Verfahren nach Anspruch 28, wobei das Gegendruckpolster aus Silikon oder Neopren besteht.

30. Verfahren nach Anspruch 28 oder 29, wobei die Fläche des zum Anstoßen an die Rückschicht bestimmten Gegendruckpolsters in die Gegenrichtung zu der Laminierungsrichtung gewölbt oder konvex ist.

## Revendications

1. Plaque (1) permettant de fixer par adhésion un appareillage de stomie sur la surface cutanée péristomiale entourant la stomie d'un sujet portant ledit appareillage, ladite plaque comprenant une couche adhésive moulée d'un seul tenant (2) constituée d'une matière de protection cutanée dilatable et absorbant l'humidité et ayant une surface distale sensiblement plane recouverte d'une couche de renfort souple (4), s'étendant au moins jusqu'au périmètre externe de la couche adhésive (2), la forme dudit périmètre externe consistant en des portions extérieurement convexes ou sensiblement rectilinéaires, pour fixer ladite plaque à une poche collectrice, et une surface proximale côté corps dont le contour est tel que ladite couche adhésive (2) comporte une portion périphérique relativement mince (9) et une portion centrale (10) relativement épaisse entourant une ouverture destinée à recevoir ladite stomie, ladite portion centrale (10) étant répartie sensiblement uniformément autour de ladite ouverture **caractérisée en ce que** ladite portion centrale (10) a une épaisseur maximale qui dépasse d'au moins 2,5 mm l'épaisseur maximale de ladite portion périphérique, dans laquelle la couche de renfort (4) inclut une languette de préhension ou une portion de préhension (7) définie par une portion de la couche de renfort non recouverte de la couche adhésive (2), et qui résulte d'un renfoncement (8) formé dans le périmètre externe de la couche adhésive (2) sur la couche de renfort (4) dépourvue de renfoncement.

2. Plaque selon la revendication 1, dans laquelle ladite portion périphérique a une largeur d'au moins 10 mm, de préférence d'au moins 12 mm et de manière préférée entre toutes d'au moins 15 mm.

3. Plaque selon la revendication 1 ou 2, dans laquelle l'épaisseur de ladite portion périphérique est au moins 0,4 mm, de préférence au moins 0,5 mm, de manière davantage préférée au moins 0,6 mm, de manière encore davantage préférée au moins 0,7 mm et de manière préférée entre toutes au moins 0,8 mm.

4. Plaque selon l'une quelconque des revendications précédentes, dans laquelle ladite portion centrale est annulaire.

5. Plaque selon la revendication 4, dans laquelle ladite ouverture est sensiblement circulaire et concentrique avec ladite portion centrale annulaire.

6. Plaque selon l'une quelconque des revendications 1 à 5, dans laquelle ladite portion centrale a une largeur radiale d'au moins 6 mm, de préférence d'au moins 7 mm et de manière préférée entre toutes supérieure ou égale à 7,5 mm.

7. Plaque selon l'une quelconque des revendications 1 à 6, dans laquelle ladite portion centrale a une largeur radiale inférieure à 11 mm, de préférence inférieure à 10 mm, de manière préférée entre toutes inférieure ou égale à 9,5 mm.

8. Plaque selon l'une quelconque des revendications précédentes, dans laquelle ladite portion centrale a une épaisseur sensiblement uniforme.

9. Plaque selon l'une quelconque des revendications 1 à 7, dans laquelle ladite portion centrale a une épaisseur qui varie dans la direction circonférentielle.

10. Plaque selon l'une quelconque des revendications 1 à 7 ou 9, dans laquelle ladite portion centrale a une épaisseur qui varie dans la direction radiale.

11. Plaque selon l'une quelconque des revendications précédentes, dans laquelle ladite surface proximale est recouverte d'une pellicule de protection souple fixée de manière amovible à ladite surface proximale, ladite pellicule de protection s'étendant au moins jusqu'au périmètre externe de ladite couche adhésive.

12. Plaque selon la revendication 11, dans laquelle une zone marginale de ladite couche adhésive faisant au moins 3 mm de large, de préférence au moins 6 mm de large et s'étendant tout le long et adjacente à toute la longueur dudit périmètre externe est conçue de manière à ce que l'angle formé entre toutes les portions mutuellement opposées de ladite pellicule de protection et de ladite couche de renfort souple recouvrant ladite zone marginale soit inférieur à 45 degrés, de préférence inférieur à 35 degrés, de manière davantage préférée inférieur à 30 degrés, de manière encore davantage préférée inférieur à 25 degrés et de manière préférée entre toutes inférieur à 20 degrés.

13. Plaque selon la revendication 12, dans laquelle l'épaisseur de ladite couche adhésive au niveau dudit périmètre externe est inférieure à 0,5 mm, de préférence inférieure à 0,4 mm et de manière préférée entre toutes inférieure à 0,3 mm.

14. Plaque selon la revendication 12, dans laquelle une zone marginale de ladite couche adhésive faisant au moins 3 mm de large, de préférence au moins 6 mm de large et s'étendant tout le long et adjacente à toute la longueur dudit périmètre externe est conçue de manière à ce que lesdites surfaces proximale et distale de ladite zone marginale diminuent l'une face à l'autre en direction dudit périmètre externe, et l'épaisseur de ladite couche adhésive au niveau dudit périmètre externe est inférieure à 0,5 mm, de préférence inférieure à 0,4 mm et de manière préférée entre toutes inférieure à 0,3 mm.

15. Plaque selon l'une quelconque des revendications précédentes, dans laquelle une zone épaissie de ladite portion périphérique relativement mince de ladite couche adhésive a une épaisseur sensiblement supérieure à l'épaisseur maximale du reste de ladite portion périphérique.

16. Plaque selon la revendication 15, dans laquelle ladite zone épaissie est située entre ladite portion centrale relativement épaisse et le périmètre externe de ladite couche adhésive.

17. Plaque selon la revendication 15 ou 16, dans laquelle l'épaisseur de ladite zone épaissie est uniforme et est comprise entre 30 % et 50 % de l'épaisseur maximale de ladite portion centrale.

18. Jeu de deux plaques au moins permettant de fixer par adhésion un appareillage de stomie sur la surface cutanée péristomiale entourant la stomie d'un sujet portant ledit appareillage, chaque plaque dudit jeu étant conforme aux revendications 1 à 17 et comprenant une couche adhésive constituée d'une matière de protection cutanée dilatable et absorbant l'humidité et ayant une surface distale sensiblement plane recouverte d'une couche de renfort souple, s'étendant au moins jusqu'au périmètre externe de la couche adhésive, pour fixer lesdites plaques à une poche collectrice, et une surface proximale côté corps dont le contour est tel que ladite couche adhésive comporte une portion périphérique relativement mince et une portion centrale relativement épaisse entourant l'ouverture destinée à recevoir ladite stomie, ladite portion centrale étant répartie sensiblement uniformément autour de ladite ouverture et ayant une épaisseur maximale qui dépasse d'au moins 2,5 mm l'épaisseur maximale de ladite portion périphérique, chacune desdites plaques dudit jeu étant différente en ce que la topographie de la surface de ladite portion centrale est différente.

19. Jeu de plaques selon la revendication 18, dans lequel au moins deux des plaques du jeu sont conformes à l'une quelconque des revendications 2 à 17.

20. Jeu de plaques selon la revendication 18 ou 19, dans lequel au moins deux plaques diffèrent l'une de l'autre en ce que la taille et/ou la forme de ladite stomie recevant l'ouverture est différente.

21. Procédé de fabrication d'une plaque permettant de fixer par adhésion un appareillage de stomie sur la surface cutanée péristomiale entourant la stomie d'un sujet portant ledit appareillage, ladite plaque comprenant une couche adhésive moulée d'un seul tenant constituée d'une matière de protection cutanée dilatable et absorbant l'humidité et ayant une surface distale sensiblement plane recouverte d'une couche de renfort souple, s'étendant au moins jusqu'au périmètre externe de la couche adhésive, la forme dudit périmètre externe consistant en des portions extérieurement convexes ou sensiblement rectilinéaires, pour fixer ladite plaque à une poche collectrice, et une surface proximale côté corps ayant une première topographie de surface telle que ladite couche adhésive comporte une portion périphérique relativement mince et une portion centrale relativement épaisse entourant l'ouverture destinée à recevoir ladite stomie, ladite surface proximale étant recouverte d'une couche de protection et ladite portion centrale ayant une épaisseur maximale qui dépasse d'au moins 2,5 mm l'épaisseur maximale de ladite portion périphérique, dans lequel la couche de renfort (4) inclut une languette de préhension (6) ou une portion de préhension (7) définie par une portion de la couche de renfort non recouverte de la couche adhésive (2), et qui résulte d'un renfoncement (8) formé dans le périmètre externe de la couche adhésive (2) sur la couche de renfort (4) dépourvue de renfoncement, le procédé consistant à : fournir un premier moule pour préformer ladite couche de protection et doté d'une première cavité de moulage ayant une seconde topographie de surface similaire à, de préférence sensiblement identique à, ladite première topographie de surface, ladite cavité étant pourvue d'ouvertures d'aspiration communiquant avec une source de vide ; fournir un second moule pour mouler ladite couche adhésive ayant une première partie et une seconde partie conçues pour être pressées l'une contre l'autre, ladite première partie étant pourvue d'une seconde cavité de moulage pour définir ladite première topographie de surface de ladite couche adhésive ; fournir un apport sensiblement continu d'une bande sacrificielle ; disposer ladite matière de protection cutanée sur ladite bande sacrificielle ; fournir un apport sensiblement continu d'une bande de protection ; chauffer une zone de ladite bande de protection pour en améliorer la déformabilité ; placer ladite zone chauffée en concordance avec ladite première cavité de moulage ; appliquer du vide auxdites ouvertures d'aspiration de manière à ce que ladite zone soit aspirée dans ladite cavité et déformée de manière à ce que ladite zone soit pourvue d'une troisième topographie de surface sensiblement identique à ladite seconde topographie de surface ; cesser ladite application de vide ; déplacer ladite zone en concordance avec ladite seconde cavité de moulage de manière à ce que ladite troisième topographie de surface avec ladite seconde topographie de surface ; déplacer une portion de ladite bande sacrificielle recouverte de la matière de protection en concordance avec ladite seconde cavité de moulage de manière à ce que ladite zone soit située entre ladite portion et ladite seconde cavité de moulage ; et presser ladite seconde partie contre ladite première partie de manière à ce que ladite zone et ladite portion soient poussées dans ladite seconde cavité de moulage.

22. Procédé de stratification d'une plaque selon l'une quelconque des revendications 1 à 17, dans lequel ledit procédé comprend les étapes suivantes consistant à : appliquer ladite couche de renfort souple sur ladite surface distale ; appliquer une pellicule de protection souple sur ladite surface proximale ; fournir un tampon ou un bloc de stratification résistant conçu pour se mouvoir dans un sens et dans l'autre dans une direction de stratification et pour exercer une pression de stratification dans ladite direction de stratification ; fournir une surface plane de contre-pression à angles sensiblement droits par rapport à ladite direction de stratification ; placer ladite plaque sur ladite surface de contre-pression avec ladite couche de renfort plane butant contre ladite surface de contre-pression ; et déplacer ledit tampon de stratification dans ladite direction de stratification de manière à ce que ledit tampon de stratification exerce une pression de stratification sur la totalité de la surface distale de ladite plaque.

23. Procédé selon la revendication 22, dans lequel la surface dudit tampon de stratification destinée à exercer ladite pression de stratification sur ladite plaque forme un dôme ou est convexe dans ladite direction de stratification.

24. Procédé selon la revendication 22 ou 23, dans lequel ledit tampon de stratification est en silicone.

25. Procédé selon l'une quelconque des revendications 22 à 24, dans lequel ledit tampon de stratification est un tampon à caractères conçu pour être imprimé sur une surface.

26. Procédé selon l'une quelconque des revendications 22 à 25, dans lequel ledit tampon de stratification a une dureté comprise entre Shore A3 et Shore A9, de préférence entre Shore A5 et Shore A7.

27. Procédé selon l'une quelconque des revendications 22 à 26, dans lequel ladite surface de contre-pression est conçue pour se mouvoir dans un sens et dans l'autre dans ladite direction de stratification.

28. Procédé selon l'une quelconque des revendications 22 à 27, dans lequel ladite surface de contre-pression est la surface d'un tampon de contre-pression résistant.

29. Procédé selon la revendication 28, dans lequel ledit tampon de contre-pression est en silicone ou en néoprène.

30. Procédé selon la revendication 28 ou 29, la surface dudit tampon de contre-pression destinée à buter contre ladite couche de renfort formant un dôme ou étant convexe dans la direction opposée à ladite direction de stratification.
